# EUROPÄISCHE PATENTSCHRIFT

(11) **EP 1 121 161 B1**
(45) Veröffentlichungstag und Bekanntmachung des Hinweises auf die Patenterteilung: **20.10.2004**
(21) Anmeldenummer: 00958445.9
(22) Anmeldetag: 14.08.2000
(51) Int. Cl.: A61L 24/00, A61L 27/10, A61L 27/12, A61K 6/033

(54) **ZUSAMMENSETZUNG ZUR IMPLANTATION IN DEN MENSCHLICHEN UND TIERISCHEN KÖRPER**
COMPOSITION FOR IMPLANTATION IN THE HUMAN AND ANIMAL BODY
COMPOSITION POUR IMPLANTATION DANS LE CORPS D'UN HOMME OU D'UN ANIMAL

(30) Priorität: 14.08.1999 DE 19938704
(43) Veröffentlichungstag der Anmeldung: 08.08.2001
(73) Patentinhaber: Ivoclar Vivadent AG, 9494 Schaan (LI)
(72) Erfinder: ROESSLER, Ralf, 35578 Wetzlar (DE)
(74) Vertreter: UEXKÜLL & STOLBERG
(86) Internationale Anmeldenummer: PCT/EP2000/007915
(87) Internationale Veröffentlichungsnummer: WO 2001/012242

(56) Entgegenhaltungen:
- EP-A- 0 543 765
- EP-A- 1 002 513
- WO-A-99/17710
- FR-A- 2 772 746
- C. KNABE, R. GILDENHAAR: "Morphological evaluation of osteoblasts cultured on different calcium phosphate ceramics" BIOMATERIALS, Bd. 18, - 1997 Seiten 1339-1347, XP002155198
- BERGER,G.; GILDENHAAR,R.: "Resorbable glass ceramics with controlled dissolution" VORTR. POSTER-SYMP. MATERIALFORSCHUNG, Bd. 3, - 1991 Seiten 2621-2623, XP000972626
- MULLER-MAI,C; BERGER,G.; VOIGT,C.: "The bony reaction to rapidly degradable glass-ceramics based on the new phase Ca2KNa(PO4)2" BIOCERAMICS, Bd. 10, - 1997 Seiten 53-56, XP000972623

## Beschreibung

Die Erfindung betrifft Zusammensetzungen zur Implantation in den menschlichen und tierischen Körper.

Diese implantierbaren Zusammensetzungen dienen insbesondere zum Ersatz oder der Reparatur von Knochen oder Zähnen sowie Teilen davon. Besonders bevorzugt werden sie im dentalen Bereich, d.h. bei Zähnen und den Knochen des Kauapparates, und daher als Dentalmaterialien eingesetzt. Die Zusammensetzungen bestehen insbesondere aus Pulvermischungen und Anmischflüssigkeiten, die unmittelbar vor der Anwendung, d. h. der Implantation, zu aushärtenden Pasten vermischt werden. Ein wesentlicher Bestandteil der Pulvermischung bildet ein calciumphosphathaltiges Pulver unterschiedlicher Zusammensetzung. Die Anmischflüssigkeit ist z.B. demineralisiertes Wasser oder eine wässrige Lösung, mit Zusatz von Stoffen, u. a. zur Verbesserung der biologischen Abbaubarkeit und/oder der Beschleunigung der Gewebeintegration. Den Zusammensetzungen können auch Antibiotika und/oder Desinfektiva zur Vermeidung von Infektionen beigemischt werden.

Ziel der vorliegenden Erfindung ist es, die Nachteile zu vermeiden, die bei den Implantationssystemen für Knochenersatz entsprechend dem Stand der Technik vorhanden sind. Diese Nachteile sind wie folgt:

Bei der Verwendung von Knochenimplantaten aus festem Material ist folgendes nachteilig:
- Es ist eine aufwendige Anpassung am Implantat oder am Knochengewebe erforderlich, damit die auszufüllende Lücke im Knochengewebe vollständig geschlossen wird.

Bei der Verwendung von Implantaten, die zu Pasten angemischt werden können, ergibt sich der vorgenannte Nachteil zwar nicht, dafür sind hier aber eine Reihe anderer Nachteile wie folgt gegeben:
- Die Aushärtezeiten der angemischten Pasten sind teilweise sehr kurz, was bei komplizierten Operationen sehr hinderlich ist. Es gibt auch Pasten mit sehr langen Aushärtezeiten, was ebenfalls unerwünscht ist.
- Die Pasten sind nach dem Einbringen in das Knochengewebe und vor dem Aushärten zum Teil nicht beständig gegen Flüssigkeiten, wie z. B. Blut, d. h sie nehmen Flüssigkeit auf und erweichen.
- Die Festigkeiten der ausgehärteten Pasten sind zum Teil nur gering. Insbesondere die Anfangsfestigkeit ist in vielen Fällen zu niedrig.
- Bei den bekanntgewordenen Implantatmaterialien auf Pastenbasis ist die Geschwindigkeit, mit der die Gewebeintegration und Resorption erfolgt, nur klein.
- Die biologische Abbaubarkeit solcher Implantate ist entweder gar nicht gegeben oder nur gering.

Details hierzu ergeben sich aus dem nachstehend beschriebenen Stand der Technik.

Das im menschlichen und tierischen Körper vorkommende mineralische Knochenmaterial besteht in seinem überwiegenden Anteil aus einer hydroxylapatitartigen Struktur, wobei die im wesentlichen vorkommenden Elemente Calcium und Phosphor sind. Es kommen jedoch auch andere Elemente wie Natrium, Kalium, Magnesium und Barium in der Knochensubstanz vor.

Der Ersatz von verlorenem Knochengewebe durch natürliche Knochen stellt auch heute noch erhebliche Probleme dar. Insbesondere in der Chirurgie des Kiefers, in der Unfallchirurgie sowie in der Orthopädie trifft dies zu. Diese Probleme resultieren aus der begrenzten Verfügbarkeit von autologem Knochen bzw. dem großen Aufwand bei seiner Gewinnung oder der unzureichenden Qualität bei Verwendung von allogenem Knochen, verbunden mit dem Risiko der Übertragung von Krankheiten.

Aber auch die derzeit als feste Substanz verfügbaren synthetischen Knochenersatzmaterialien wie Bio-Oss®, Synthacer® oder die Biogläser haben erhebliche Nachteile. Diese bestehen z. B. darin, daß sie aufgrund ihrer präformierten, granulären Struktur nicht optimal in die knöchernen Defekte zu implantieren sind. Dies gilt insbesondere in der Zahnmedizin wegen der beengten Platzverhältnisse in der Mundhöhle, wo präformierter Knochenersatz nur sehr schwer zu plazieren ist. Ein weiterer Nachteil dieser zum Teil gesinterten Materialien besteht darin, daß diese zwar knöchern integriert, aber nicht vollständig abgebaut und durch natürlichen Knochen ersetzt werden.

Seit einiger Zeit werden jedoch auch calciumphosphathaltige Pulver benutzt, welche nach Anmischen mit Wasser oder wässrigen Lösungen zunächst als Paste vorliegen und nach einer gewissen Zeit aushärten. Nach der Aushärtung haben diese Materialien im Röntgendiffraktogramm eine strukturelle Ähnlichkeit mit der mineralischen Phase von natürlichem Knochen. Probleme mit dem Einpassen in knöcherne Defekte des Patienten gibt es damit nicht mehr, da sich die Pasten den vorhandenen Strukturen genau anpassen.

Nachteile bei den bekanntgewordenen Implantaten auf Pastenbasis, entsprechend dem Stand der Technik, ergeben sich bei den Aushärtezeiten, der Beständigkeit gegen von außen wirkende Flüssigkeiten, der Festigkeit nach dem Aushärten, der Gewebeintegration sowie bei der biologischen Abbaubarkeit. Im Detail wird auf diese Themen nachstehend, bei der weiteren Beschreibung des Standes der Technik, noch eingegangen.

Eines der ersten Implantate auf Pulver- bzw. Pastenbasis ist das von Brown und Chow 1965 erfundene BoneSource®, bei welchem die Pulverkomponente aus CaHPO₄ und Tetracalciumphosphat besteht. Nach Anmischen mit Wasser entsteht daraus eine aushärtende Zementpaste, deren ausgehärtetes Endprodukt ein nanokristalliner Hydroxylapatit mit einem Calcium/Phosphor-Verhältnis (Ca/P) von 1,67 ist (d.h. auf 1,67 Calciumatome kommt ein Phosphoratom). Nachteil eines solchen Calciumphosphat-Zementes ist seine relativ lange Aushärtedauer und die unvorteilhafte Eigenschaft, in der Frühphase des Abbindens zu zerfallen, wenn direkter Kontakt mit Flüssigkeiten stattfindet. Dies gilt insbesondere beim direkten Einbringen in blutende Knochenwunden.

In der Publikation von Driessens et al, Bioceramics Band 9, 1996, Seiten 231-234, werden amorphe Calciumphosphatzemente beschrieben deren Calcium/Phosphor-Verhältnis (Ca/P) < 1 ist. Diese Zemente bestehen aus Mischungen von Ca(H₂PO₄)₂·H₂O = Mono-Calciumphosphat-Monohydrat (MCPM) und CaKPO₄ oder Mischungen von MCPM und Ca₂NaK(PO₄)₂. Sie besitzen infolge ihrer relativ hohen Löslichkeit in Körperflüssigkeiten geringere Druckfestigkeiten als die vorher beschriebenen Zemente mit hohem Ca/P Verhältnis. Die verminderte Druckfestigkeit ist ein Nachteil bei diesen Implantationsmaterialien.

Erwünscht ist hingegen eine gewisse, wenn auch geringe, Löslichkeit solcher Materialien in den Körperflüssigkeiten, da die Materialien dann schneller durch Körperzellen erschlossen werden können, als dies bei Zementen der Fall ist, die in menschlichen bzw. in tierischen Körperflüssigkeiten unlöslich sind und im wesentlichen als osteotransduktiv bezeichnet werden müssen.

Das Systems aus MCPM und CaKPO₄ hat außer dem Nachteil seiner geringen Druckfestigkeit nach dem Aushärten auch den Nachteil, daß seine Verarbeitungszeit weniger als 3 Minuten beträgt.

Diese Verarbeitungszeit ist bei schwierigen Implantat-Operationen eindeutig zu kurz. Das ebenfalls erwähnte System MCPM und Ca₂NaK(PO₄)₂ weist zwar höhere Druckfestigkeiten auf, die Aushärtedauer ist im Vergleich zum System aus MCPM und CaKPO₄ jedoch deutlich zu lang.

Solche Zementsysteme und die nach Aushärtung daraus hervorgehenden Produkte wie Ca(H₂PO₄)₂·H₂O (= MCPM), Hydroxylapatit Ca₁₀(PO₄)₈(OH)₂, und andere sind aus den Patenten US 4,673,355, US 5,053,212 oder EP 0543765 bekannt. Die Resorbierbarkeit dieser Materialien sowie die Aushärtedauer sind, wie erwähnt, jedoch noch nicht zufriedenstellend.

Aus diesem Grund liegt der Erfindung die Aufgabe zugrunde, neue implantierbare Zusammensetzungen zu entwickeln, mit denen aushärtbare, calciumphosphathaltige Pasten angemischt werden können. Wichtig waren verbesserte biologische Eigenschaften für den klinischen Erfolg am Patienten, aber auch günstigere Handlings-Eigenschaften für den behandelnden Arzt, z.B. bezüglich der Aushärtezeit. Die erfindungsgemäße Zusammensetzung erfüllt diese Forderungen in idealer weise.

Sie bietet insbesondere die folgenden Vorteile:
- Da sie zu aushärtenden Pasten angemischt werden kann, ergibt sich eine ideale Anpassbarkeit an die beim Patienten vorhandene Knochenstruktur.
- Durch Variation der Zusammensetzung kann die Ausnärtezeit verändert werden, was bei der Anwendung außerordentlich vorteilhaft ist.
- Während des Aushärtens ist die Zusammensetzung beständig gegen von außen einwirkende Flüssigkeiten, wie z.B. Blut, das bei blutenden Knochenwunden auftreten kann.
- Nach dem Aushärten der Implantatpaste ergibt sich sehr schnell eine hohe Anfangsfestigkeit des Implantats.
- Durch Zusatzstoffe kann die Gewebeintegration wesentlich beschleunigt werden
- Die besondere Art der Zusammensetzung führt zu einer guten biologischen Abbaubarkeit, deren Geschwindigkeit durch die beigemischten Additive beeinflußbar ist.

Bei den Implantaten entsprechend dem Stand der Technik sind diese positiven Eigenschaften in Kombination miteinander nicht vorhanden, so daß die erfindungsgemäße implantierbare Zusammensetzung einen erheblichen technischen Fortschritt darstellt.

Die erfindungsgemäße implantierbare Zusammensetzung zeichnet sich dadurch aus, dass sie eine Basismischung aus
(a) CaKPO₄
(b) Ca₂NaK(PO₄)₂ und
(c) Ca(H₂PO₄)₂·H₂O
enthält. Die als Basismischung bezeichnete Kombination von (a), (b) und (c) liegt insbesondere in Form einer Pulvermischung vor.

Gegenstand der vorliegenden Erfindung ist weiter die Zusammenstellung geeigneter Mischungen von Calcium und Phosphor enthaltenden Pulvern mit verschiedenen pulverförmigen Additiven und Anmischflüssigkeiten, die aus Wasser oder wässrigen Lösungen mit verschiedenen Zusatzstoffen bestehen.

Hauptmerkmal bei den Calcium und Phosphor enthaltenden erfindungsgemäßen Pulvermischungen ist, daß sie als wesentlichen Bestandteil spezielle Mischungen von CaKPO₄, Ca₂NaK(PO₄)₂ und Ca(H₂PO₄)₂·H₂O (=MCPM) enthalten, die als Basismischungen bezeichnet werden. Diesen Basismischungen werden vorzugsweise weitere calciumphosphathaltige Pulver beigemischt. Es ist jedoch auch die Beimengung anderer Stoffe vorgesehen. Mit den erfindungsgemäßen Zusammensetzungen werden die vorstehend genannten Nachteile bei den Zementsystemen entsprechend dem Stand der Technik vermieden.

Eine bevorzugte Pulvermischung der erfindungsgemäßen Zusammensetzung besteht aus der Basismischung mit Beimischungen von CaHPO₄·H₂O und/oder CaCO₃, wobei noch weitere variable Anteile anderer calciumphosphathaltiger Substanzen und andere Additive beigemischt sein können. Durch Variation der Mengenverhältnisse der einzelnen genannten Komponenten lassen sich die Eigenschaften der erfindungsgemäßen Zusammensetzung in einem weiten Bereich verändern und somit an den jeweiligen Bedarfsfall anpassen.

Weiter enthält die erfindungsgemäße Zusammensetzung vorzugsweise eine Anmischflüssigkeit, die insbesondere wasserhaltig sein kann. Mit dieser Flüssigkeit kann die Zusmmensetzung zu einer Paste verarbeitet werden, die auf das zu behandelnde Substrat aufgebracht, geformt und schließendlich gehärtet wird.

Die zum Anmischen und Aushärten verwendeten Anmischflüssigkeiten können, wie erwähnt, Zusatzstoffe enthalten, mit denen die positiven Eigenschaften der Zusammensetzung ebenfalls entscheidend beeinflußt werden können. Dies gilt z.B. bezüglich der biologischen Zugänglichkeit des ausgehärteten Zementes für die körpereigenen Zellen, da hiervon ganz wesentlich die Gewebeintegration abhängt.

Die Anmischflüssigkeit enthält bei einer bevorzugten Rezeptur eine wässrige Lösung von Mannose-6-Phosphat (M-6-P). Bei einigen Rezepturen ist auch vorgesehen, daß der Anmischflüssigkeit Saccharose-Octasulfat beigemischt wird. Es sind auch Mischungen von Mannose-6-Phosphat und Saccharose-Octasulfat in der Anmischflüssigkeit vorgesehen. Die beiden genannten Stoffe können jedoch bei bestimmten Rezepturen auch der Pulverkomponente zugemischt werden. In einer bevorzugten Rezeptur wird das Saccharose-Octasulfat als Natrium- oder Kaliumkomplexsalz verwendet.

Es ist auch möglich, daß der Anmischflüssigkeit Antibiotika und Desinfektiva beigemischt werden, damit es nicht zu Infektionen kommt, solange das Implantat noch nicht in das Gewebe integriert ist. Durch die Implantation von zunächst noch unbelebtem Calcium-Phosphatzement besteht immer das Risiko der Besiedlung mit Keimen, da der Zement noch nicht resorbiert und/oder zellulär bzw. vaskulär erschlossen ist. Es besteht deshalb eine begründete Forderung von der Anwenderseite, solche Materialien mit Stoffen zu versehen, welche eine Keimbesiedelung hemmen oder durch Abgabe keimhemmender oder -abtötender Wirkstoffe in die Implantatumgebung, diese vor Keimbesiedlung schützen oder die Umgebung durch Freisetzung von Medikamenten im Sinne eines Drug Delivery Systems bzw. Wirkstoffdepots therapieren.

Dies wird dadurch erzielt, daß dem Zementpulver Antibiotika oder andere keimhemmende und/oder abtötende Stoffe wie z.B. Biguanide (z.B. Chlorhexidin) zugemischt werden. Die Antibiotika und/oder Desinfektiva können aber auch der Anmischflüssigkeit, die zum Aushärten des Zementpulvers verwendet wird, beigegeben werden.

Es ist auch möglich, Antibiotika enthaltende bzw. desinfizierende wässrige Lösungen, wie sie auch kommerziell erhältlich sind, als Anmischflüssigkeiten zu verwenden. In diesem Zusammenhang werden vorzugsweise folgende Stoffe benutzt:

Gentamicin enthaltende Injektionslösungen, wie z.B. Duragentamicin 80 und Duragentamicin 160 (von Fa. Durachemie), Tobramycin-Lösungen oder Clindamycin enthaltende Lösungen, wie z.B. Sobelin Solubile 300, 600 und 900mg, oder Metronidazol enthaltende Lösungen, wie z.B. Clont i.v. (von Fa. Bayer) oder auch Metronidazol i.v. (von Fa. Braun). Als desinfizierende Lösung ist eine mit NaCl angesetzte Lavasept Lösung (von Fa. Fresenius) ebenfalls geeignet.

Die erfindungsgemäße Zusammensetzung hat den Vorteil, daß sich insbesondere durch Variation der Mengenverhältnisse von CaKPO₄ und Ca₂NaK(PO₄)₂ zusammen mit MCPM und ggf. vorhandenen, CaHPO₄·H₂O die Aushärtegeschwindigkeit und die Druckfestigkeit der ausgehärteten Masse im gewünschten Sinne beeinflußbar ist. Hohe Anteile von CaKPO₄ und MCPM ergeben kurze Aushärtezeiten mit einer etwas geringeren Druckfestigkeit. Umgekehrt läßt sich durch höhere Anteile von Ca₂NaK(PO₄)₂ und MCPM eine größere Druckfestigkeit erzielen, wobei sich etwas längere Aushärtezeiten ergeben.

Weitere Möglichkeiten zur Einflußnahme auf die Systemeigenschaften ergeben sich durch die Zugabe variabler Anteile von CaCO₃ und/oder nanopartikulärem Hydroxylapatit (entsprechend der EP 0 664 133 A1). Höhere Anteile von nanopartikulärem Hydroxylapatit ergeben die gewünschte gute biologische Abbaubarkeit der ausgehärteten Zusammensetzung im Körper des Patienten. Höhere Anteile von CaCO₃ führen zu einer hohen Festigkeit nach dem Aushärten, wobei sich die Aushärtezeit etwas verlängert.

Ferner zeigte sich, daß die Aushärtegeschwindigkeit der Pulver-/Flüssigkeitsmischung durch die Zugabe von wässriger Na₂HPO₄-Lösung mit Konzentrationen von insbesondere bis zu 5% deutlich ansteigt, so daß der Operateur die Möglichkeit hat, eine für den vorliegenden Fall optimale Aushärtegeschwindigkeit auszuwählen.

Aus der Publikation von Martinez et al, J-Cell-Biochem, 59(2), 1995, geht hervor, daß Osteoblasten einen Rezeptor für Mannose-6-Phosphat (M-6-P) besitzen, wobei durch intrazelluläre Signalübermittlung die Knochenneubildung durch Osteoblasten stimuliert wird. Von Young et al., Invest. Radiol. 26(5), 1991, wurde über eine verbesserte Frakturheilung bei Kaninchen durch die Gabe von Saccharose-Octasulfat berichtet. Die Wirkung des Saccharose-Octasulfates soll dabei über die Bindung lokal vorhandener Wachstumsfaktoren wie EGF und FGF (Szabo et al, Scand-J-Gastroenterol, 185, 1991 ) und/oder Stimulation des endogenen Prostaglandinsystems erfolgen (Stern et al, Am-J-Med, 83(3B), 1987). Die positive Wirkung des Saccharose-Octasulfates auf das Knochenwachstum wurde in der Literatur bisher jedoch noch nicht im Zusammenhang mit Reaktionssystemen für Knochenersatz erwähnt. Insofern ist die Zugabe dieses Stoffes zu der erfindungsgemäßen Zusammensetzung eine bevorzugte Ausführungsform.

Ein weiteres wichtiges Merkmal der vorliegenden Erfindung bezieht sich auf die Kombination von M-6-P und Saccharose-Octasulfat als Additive für die Basismischung (CaKPO₄ Ca₂NaK(PO₄)₂, MCPM) in Kombination mit CaHPO₄·H₂O und CaCO₃. Das Saccharose-Octasulfat kann in Form einer wässrigen Lösung als flüssige Komponente in den Zement eingebracht oder in Pulverform der mineralischen Pulverkomponente zugefügt werden. Dadurch wird die Aufgabe gelöst, einen Calciumphosphat-Zement mit den erwähnten anwenderoptimierten Aushärteeigenschaften und guter Festigkeit nach dem Aushärten zusammenzustellen, der gleichzeitig über die Fähigkeit verfügt, Wachstumsfaktoren zu binden.

Dadurch wird ein zellulärer Aufschluß des Implantats über die Neubildung von Blutgefäßen ermöglicht. Zusätzlich werden noch Osteoblasten zur Neubildung von Knochen stimuliert.

Durch diese Kombination wird eine verstärkte biologische Aktivität und damit auch Resorbierbarkeit des synthetischen Knochenersatzmaterials erreicht. Die Beständigkeit gegen Flüssigkeiten, wie z.B. Blut, ergibt sich aus der Kohäsion der benutzten Komponenten.

Als besonders geeignete Rezepturen für die Basismischung (CaKPO₄, Ca₂NaK(PO₄)₂ und MCPM) haben sich Mischungen mit folgenden relativen Gewichtsanteilen herausgestellt:
- Der Anteil von CaKPO₄ und Ca₂NaK(PO₄)₂ an der Gesamtbasismischung liegt zwischen 80% und 65%.
- Der Anteil von MCPM beträgt dementsprechend 20% bis 35%
- Das Verhältnis von CaKPO₄ zu Ca₂NaK(PO₄)₂, bezogen auf die Gesamtmenge dieser beiden Stoffe, kann dabei in weiten Grenzen variiert werden. So kann der Anteil von CaKPO₄ zwischen 1% und 99% liegen, wobei dementsprechend der Anteil von Ca₂NaK(PO₄)₂ zwischen 99% und 1% liegt.

Es ist bevorzugt, die drei Stoffe des Basisgemischs in einem ungefähren molaren Verhältnis von 2 Mol CaKPO₄, 1 Mol Ca₂NaK(PO₄)₂ und 1 Mol MCPM einzusetzen.

Die Herstellung der beiden für das erfindungsgemäße Reaktionssystem benutzten Komponenten CaKPO₄ und Ca₂NaK(PO₄)₂ wird insbesondere wie nachstehend beschrieben durchgeführt.

### Herstellung von CaKPO₄

Zunächst wird 1 Mol K₂CO₃ mit 2 Molen CaHPO₄ gemischt, und die Pulvermischung wird bei 1000°C ca. 1 Stunde lang gebrannt. An das Brennen schließt sich ein rasches Abkühlen auf Temperaturen <700°C an. Die Abkühlzeit darf nicht größer als 1 Minute sein.

### Herstellung von Ca₂NaK(PO₄)₂

Das Ca₂NaK(PO₄)₂ wird analog zu dem CaKPO₄ hergestellt, d.h. es werden für das Brennen und das Abkühlen die gleichen Verfahrensparameter benutzt. Die Pulvermischung vor dem Brennen besteht aus 1 Mol K₂CO₃, 1 Mol Na₂CO₃ und 4 Molen CaHPO₄.

Es ist bevorzugt, dass die in der erfindungsgemäßen Zusammensetzung enthaltenen Komponenten CaKPO₄ und Ca₂NaK(PO₄)₂ bei 1000°C gebrannt und dann durch schnelles Abfüllen innerhalb von 1 Minute auf weniger als 700°C gebracht werden.

Um die biologischen Eigenschaften der erfindungsgemäßen Zusammensetzung weiter zu verbessern, können sowohl den Pulvermischungen, als auch den Anmischflüssigkeiten biologisch aktive Proteine wie Wachstumsfaktoren zugemischt werden. In Frage kommen z.B. Fibroblasten Wachstumsfaktor (FGF), knochenmorphogenetische Proteine (BMP's), Schmelzamelogenine oder Elastase-Inhibitoren wie z. B. AEBSF (P 2714, von Fa. Sigma). Knochenmorphogenetische Proteine sind Substanzen, die den Proteinstoffwechsel bei der Synthese von neuem Gewebe in Richtung einer bestimmten Strukturklasse, hier in Richtung Knochenwachstum beeinflussen. Schmelzamelogenine sind ebenfalls knochenmorphogenetische Proteine, die speziell für das Wachstum des Zahnhalteapparates zuständig sind.

Die erfindungsgemäßen Zusammensetzungen aus Pulvermischungen und Anmischflüssigkeiten ergeben Implantationspasten, die sowohl bei Raum- als auch bei Körpertemperatur abbinden.

Die ausgehärteten Implantationspasten haben Kompressionsfestigkeiten die abhängig sind von ihrer Zusammensetzung und mehr als 5 MPa betragen können. Sie zeichnen sich durch eine für den Anwender in weiten Bereichen einstellbare Aushärtezeit aus. Eine besondere Ausführungsform stellt die Verwendung einer Pulvermischung nach Beispiel 2 dar, wodurch die Erschließung des zunächst zellulär unbelebten Materials durch Zellen des menschlichen oder tierischen Körpers sehr positiv beeinflußt wird.

Die Erfindung betrifft schließlich die Verwendung der Zusammensetzung zur Reparatur oder zum Ersatz von Knochen, Zähnen oder Teilen davon. Dabei wird insbesondere so vorgegangen, dass die Zusammensetzung in Form einer Paste auf das ausgewählte Substrat aufgebracht, geformt und gehärtet wird.

Verschiedene Rezepturen haben sich für die unterschiedlichen Anwendungen als besonders vorteilhaft erwiesen. Nachstehend werden einige der besonders vorteilhaften Rezepturen in den Beispielen 1 bis 10 beschrieben. Es sind jedoch auch andere Rezepturen vorgesehen.

### Beispiel 1:

Basismischung bestehend aus 13,92g CaKPO₄ und 19,93g Ca₂NaK(PO₄)₂ (entsprechend 41,1 Gew.-% bzw. 58,9 Gew.-% des Gemisches der beiden Stoffe) und 10, 41g Ca(H₂PO₄)·H₂O (=MCPM) (entsprechend 23,5 Gew.-% MCPM an der Gesamtmenge der Basismischung).

Die Pulvermischung wird intensiv vermischt, wobei eine Vorvermahlung der einzelnen Pulverbestandteile in einer Kugelmühle erfolgt. Die Aushärtecharakteristik der Zementmischung ergibt sich nach Anmischen von 2g Pulver mit 1ml Wasser in Anlehnung an die ASTM C266-89 (Norm zur Bestimmung der Verfestigung von Leim, Zement und Gips) wie folgt:
- 1.: Aushärtezeit (1.Azt), gemessen mit der leichten Gillmore-Nadel nach 4 Min. 5 Sek.
- 2.: Aushärtezeit (2.Azt), gemessen mit schwerer Gillmore-Nadel, nach 8 Min. 40 Sek.

Mit der oben beschriebenen Rezeptur und dem gleichen Pulver/-Flüssigkeitsverhältnis erreichen zylindrische Prüfkörper der Dimension Durchmesser 6mm x Höhe 12mm nach 24 stündiger Inkubation in Ringerlösung eine Druckfestigkeit von 10,6 MPa.

Diese Zusammensetzung zeigt somit eine sehr gute Reaktionszeit beim Aushärten und ergibt gute Druckfestigkeiten.

### Beispiel 2:

Basismischung hergestellt wie in Beispiel 1 beschrieben. Als Anmischflüssigkeit zur Herstellung einer implantierbaren Paste wird jedoch auf 2g Pulver 1 ml einer wässrigen Lösung aus 5mMol Mannose-6-Phosphat (M-6-P) der Firma Sigma verwendet, welche zusätzlich 100µg Saccharose-Octasulfat pro 1 ml enthält.

Die 1.Azt beträgt in diesem Fall 4 Min. und die 2.Azt 8 Min. 10 Sek.

### Beispiel 3:

Außer den in Beispiel 1 und 2 dargestellten und vorher beschriebenen Pulvermischungen werden weitere Mischungen aus der Basismischung (CaKPO₄, Ca₂NaK(PO₄)₂ und MCPM) sowie CaHPO₄·H₂O, CaCO₃ und CaHPO₄ vorgeschlagen. Als besonders geeignete Pulvermischungen erwiesen sich Kombinationen aus der Basismischung mit Beimischungen von CaHPO₄·H₂O in Mengen von 1 bis 60 Gew.-%, bezogen auf die Gesamtpulvermenge, bzw. Beimischungen von CaCO₃ in Mengen von 1 bis 60 Gew.-%, bezogen auf die Gesamtpulvermenge.

Vorteilhaft ist auch die Beimischung von Kombinationen aus CaHPO₄·H₂O und CaCO₃ zu der Basismischung, wobei der Anteil in Gewichtsprozenten von CaHPO₄·H₂O zwischen 1% bis 30% und der Anteil von CaCO₃ zwischen 30% bis 1% liegt, bezogen auf die Gesamtmischung.

### Beispiel 4:

Mischung der folgenden Zusammensetzung: Basismischung bestehend aus: 13,92g CaKPO₄ + 19,93g Ca₂NaK(PO₄)₂ + 10,41g MCPM mit Zusatz von 11,5g CaHPO₄·H₂O

Die genannten Komponenten werden in einer Kugelmühle gemischt und gemahlen. Beim Anmischen von 2g dieses Pulvers mit 0,9 ml Wasser ergibt sich eine 1.Azt von 4 Min. 30 Sek. und eine 2.Azt von 11 Min. 15 Sek.

Die Paste kann anstelle von Wasser auch mit 0,9 ml einer wässrigen, 2%igen Na₂HPO₄-Lösung angemischt werden. Die 1.Azt beträgt dann 2 Min. 45 Sek. und die 2.Azt 7 Min. 25 Sek.

Durch das Zumischen von CaHPO₄·H₂O (DCPD) erhöht sich die Kompressionsfestigkeit eines Prüf zylinders (siehe Beispiel 1) auf 16 MPa. Dies ist ein deutlich höherer Wert als in Beispiel 1 erwähnt, bei dem die Rezeptur ohne CaHPO₄·H₂O angegeben war.

### Beispiel 5:

Hier handelt es sich um eine ähnliche Pulvermischung wie in Beispiel 4 beschrieben. Anstelle des CaHPO₄·H₂O (DCPD) wird jedoch sein Anhydrid (CaHPO₄) verwendet, die übrigen Bestandteile bleiben gleich.

Bei der Anmischung von 2g des Pulvers mit 0,9ml einer wässrigen 2%igen Na₂HPO₄-Lösung werden die gleichen Aushärtezeiten erreicht, wie unter Beispiel 4 beschrieben, jedoch verringert sich die Kompressionsfestigkeit auf 6,9 MPa (gemessen wie in Beispiel 1 beschrieben).

### Beispiel 6:

Hierbei handelt es sich um eine Pulvermischung wie in Beispiel 1 beschrieben, mit einer Zugabe von 10% CaCO₃.

Die 1. Azt beträgt 2 Min, die 2.Azt beträgt 7 Min 45 Sek. Die Kompressionsfestigkeit ergibt sich zu 7,3 MPa nach Inkubation in Ringerlösung über 24 Stunden bei 37°C.

### Beispiel 7:

Bei dieser Rezeptur handelt es sich zunächst um eine Pulvermischung wie in Beispiel 1 beschrieben, jedoch mit zusätzlicher Zugabe von 6g CaHPO₄·H₂O und 6g CaCO₃. Nach Anmischen von 2g des Pulvers mit 1 ml Wasser ergibt sich eine 1.Azt von 6 Min. 30 Sek. und eine 2.Azt von 12 Minuten.

Bei Verwendung der gleichen Pulvermischung, jedoch mit einer 4%igen Na₂HPO₄ Lösung anstelle des Wassers wird eine 1.Azt von 3 Min. 30 Sek. und eine 2.Azt von 9 Min. 15 Sek. erhalten.

### Beispiel 8:

Bei dieser Rezeptur werden 2g Pulvermischung mit einer Zusammensetzung entsprechend Beispiel 1 mit 1ml Sobelin Solubile 600mg-Lösung angemischt. Die Aushärtezeiten sind im Vergleich zu den in Beispiel 1 genannten Zeiten etwas verlängert. Die 1.Azt ist 6 Min. 30 Sek. und die 2.Azt ist 13 Minuten.

### Beispiel 9:

Bei dieser Rezeptur werden 2g Pulvermischung, mit einer Zusammensetzung entsprechend Beispiel 1, mit 1ml Duragentamicin 160 mg-Lösung angemischt. Das Anmischen ergibt bereits nach 15 Sekunden eine Paste guter Konsistenz, die beim Kontakt mit weiterer Flüssigkeit nicht mehr zur Entmischung oder Aufweichung führt. Die Aushärtezeiten im Vergleich zu Beispiel 1 sind unverändert.

### Beispiel 10 :

Bei dieser Rezeptur werden 2g Pulvermischung, mit einer Zusammensetzung entsprechend Beispiel 1, mit 1ml Clont i.v. Infusionslösung (von Fa. Bayer) angemischt. Bereits 15 Sekunden nach Beginn des Anmischens wird eine geschmeidige, fest adherierende Paste erhalten, die bei Kontakt mit Wasser nicht mehr dispergiert. Die Aushärtezeiten sind im Vergleich zu Beispiel 1 etwas verkürzt. Die 1. Azt ist 3 Min 55 Sek und die 2. Azt ist 8 Min.

## Patentansprüche

1. Implantierbare Zusammensetzung, die eine Basismischung aus
(a) CaKPO₄,
(b) Ca₂NaK(PO₄)₂ und
(c) Ca(H₂PO₄)₂·H₂O
enthält.

2. Zusammensetzung nach Anspruch 1, bei der die Basismischung
- 80 bis 65 Gew.% an der Kombination von CaKPO₄ (a) und Ca₂NaK(PO₄)₂ (b) sowie
- 20 bis 35 Gew.% an Ca(H₂PO₄)₂·H₂O (c)
aufweist.

3. Zusammensetzung nach Anspruch 1 oder 2, bei der, bezogen auf die Gesamtmenge von CaKPO₄ und Ca₂NaK(PO₄)₂, der Gewichtsanteil von CaKPO₄ zwischen 1% und 99% und der von Ca₂NaK(PO₄)₂ zwischen 99% und 1% liegt.

4. Zusammensetzung nach einem der Ansprüche 1 bis 3, bei der die Basismischung in Form einer Pulvermischung vorliegt.

5. Zusammensetzung nach einem der Ansprüche 1 bis 4, die zusätzlich mindestens eine der folgenden Komponenten enthält:
CaHPO₄·H₂O, CaCO₃, CaSO₄, CaSO₄·0,5H₂O, CaSO₄·2H₂O,
CaHPO₄, nanopartikulärer Hydroxylapatit.

6. Zusammensetzung nach einem der Ansprüche 1 bis 5, die eine Pulvermischung aus der Basismischung mit Beimischung von CaHPO₄·H₂O und CaCO₃ enthält und, bezogen auf die Pulvermischung, der Gewichtsanteil von CaHPO₄·H₂O zwischen 1% und 30% und der Gewichtsanteil von CaCO₃ zwischen 30% und 1% liegt.

7. Zusammensetzung nach einem der Ansprüche 1 bis 6, die eine Pulvermischung aus der Basismischung mit Beimischung von CaHPO₄·H₂O enthält und, bezogen auf die Pulvermischung, der Gewichtsanteil von CaHPO₄·H₂O zwischen 1% und 60% liegt.

8. Zusammensetzung nach einem der Ansprüche 1 bis 7, die eine Pulvermischung aus der Basismischung mit Beimischung von CaCO₃ enthält und, bezogen auf die Pulvermischung, der Gewichtsanteil von CaCO₃ zwischen 1% und 60% liegt.

9. Zusammensetzung nach einem der Ansprüche 1 bis 8, die zusätzlich mindestens eine der folgenden Komponenten enthält:
Mannose-6-Phosphat, Saccarose-Octasulfat, Saccarose-Octasulfat in Form des Natrium- oder Kaliumkomplexsalzes, ein Antibiotikum und ein Desinfektivum.

10. Zusammensetzung nach einem der Ansprüche 1 bis 9, die zusätzlich eine Anmischflüssigkeit enthält.

11. Zusammensetzung nach Anspruch 10, bei der die Anmischflüssigkeit wasserhaltig ist.

12. Zusammensetzung nach Anspruch 10 oder 11, die in Form einer Paste vorliegt.

13. Zusammensetzung nach einem der Ansprüche 10 bis 12, bei der die Anmischflüssigkeit eine wässrige Lösung von Mannose-6-Phosphat, von Saccharose-Octasulfat oder von einer Mischung von diesen enthält.

14. Zusammenssetzung nach einem der Ansprüche 10 bis 13, bei der die Anmischflüssigkeit eine wässrige Lösung von Na₂HPO₄ enthält.

15. Zusammenssetzung nach einem der Ansprüche 10 bis 14, bei der die Anmischflüssigkeit mindestens eine der folgenden Komponenten enthält
Mannose-6-Phosphat, Saccharose-Octasulfat, Saccarose-Octasulfat in Form des Natrium- oder Kaliumkomplexsalzes, Na₂HPO₄, ein Antibiotikum und ein Desinfektivum.

16. Zusammensetzung nach einem der Ansprüche 1 bis 15, die zusätzlich biologisch aktive Proteine enthält.

17. Zusammensetzung nach Anspruch 16, die als biologisch aktive Proteine Wachstumsfaktoren, knochenmorphogenetische Proteine, Schmelzamelogenine und/oder Elastase-Inhibitoren enthält.

18. Verwendung der Zusammensetzung nach einem der Ansprüche 1 bis 17 zur Herstellung eines Implantats zur Reparatur oder zum Ersatz von Knochen, Zähnen oder Teilen davon.

19. Verwendung nach Anspruch 18, bei der die Zusammensetzung in Form einer Paste auf das ausgewählte Substrat aufgebracht, geformt und gehärtet wird.

## Claims

1. Implantable composition comprising a base mixture of
(a) CaKPO₄
(b) Ca₂NaK(PO₄)₂ and
(c) Ca(H₂PO₄)₂ .H₂O.

2. Composition according to Claim 1 in which the base mixture comprises
• 80 to 65 wt.% of the combination of CaKPO₄ (a) and Ca₂NaK(PO₄)₂(b) together with
• 20 to 35 wt.% Ca(H₂PO₄)₂ .H₂O.

3. Composition according to Claim 1 or 2, in which, relative to the overall quantity of CaKPO₄ and Ca₂NaK(PO₄)₂, the proportion by weight of CaKPO₄ lies between 1% and 99% and that of Ca₂NaK(PO₄)₂ between 99% and 1%.

4. Composition according to any one of Claims 1 to 3, in which the base material is present in the form of a powder mixture.

5. Composition according to any one of Claims 1 to 4, which in addition comprises at least one of the following components:
CaHPO₄ .H₂O, CaCO₃, CaSO₄, CaSO₄. 0.5H₂O, CaSO₄ .2H₂O, CaHPO₄, nanoparticular hydroxylapatite.

6. Composition according to any one of Claims 1 to 5, which comprises a powder mixture of the base mixture with addition of CaHPO₄ .H₂O and CaCO₃ and wherein, relative to the powder mixture, the proportion by weight of CaHPO₄ .H₂O lies between 1% and 30% and the proportion by weight of CaCO₃ between 30% and 1%.

7. Composition according to any one of Claims 1 to 5, which comprises a powder mixture of the base mixture with addition of CaHPO₄ .H₂O and wherein, relative to the powder mixture, the proportion by weight of CaHPO₄ .H₂O lies between 1% and 60%.

8. Composition according to any one of Claims 1 to 7, which comprises a powder mixture of the base mixture with addition of CaCO₃ and wherein, relative to the powder mixture, the proportion by weight of CaCO₃ lies between 1 % and 60%.

9. Composition according to any one of Claims 1 to 8, which also comprises at least one of the following components:
mannose 6-phosphate, saccharose octasulphate, saccharose octasulphate in the form of the sodium or potassium complex salt, an antibiotic and a disinfectant.

10. Composition according to any one of Claims 1 to 9, which also comprises an admixing liquid.

11. Composition according to Claim 10, in which the admixing liquid contains water.

12. Composition according to Claim 10 or 11, which is present in the form of a paste.

13. Composition according to any one of Claim 10 to 12, in which the admixing liquid comprises an aqueous solution of mannose 6-phosphate, of saccharose octasulphate or of a mixture of these.

14. Composition according to any one of Claims 10 to 13, in which the admixing liquid comprises an aqueous solution of Na₂HPO₄.

15. Composition according to any one of Claims 10 to 14, in which the admixing liquid comprises at least one of the following components
mannose 6-phosphate, saccharose octasulphate, saccharose octasulphate in the form of the sodium or potassium complex salt, an antibiotic and a disinfectant.

16. Composition according to any one of Claims 1 to 15, which also comprises biologically active proteins.

17. Composition according to Claim 16, which comprises growth factors, bone morphogenetic proteins, enamel amelogenins and/or elastase inhibitors as biologically active proteins

18. Use of the composition according to any one of the Claims 1 to 17 for the preparation of an implant for the repair or replacement of bones, teeth or parts thereof.

19. Use according to Claim 18, in which the composition is applied to the selected substrate in the form of a paste, moulded and cured.

## Revendications

1. Composition implantable, contenant un mélange de base de
(a) CaKPO₄
(b) Ca₂NaK(PO₄)₂ et
(c) Ca(H₂PO₄)₂·H₂O.

2. Composition selon la revendication 1, dans laquelle le mélange de base présente
- 80 à 65 % en poids de la combinaison de CaKPO₄ (a) et de Ca₂NaK(PO₄)₂ (b) ainsi que
- 20 à 35 % en poids de Ca(H₂PO₄)₂·H₂O (c).

3. Composition selon la revendication 1 ou 2, dans laquelle, par rapport à la quantité totale de CaKPO₄ et de Ca₂NaK(PO₄)₂, la part en poids de CaKPO₄ se situe entre 1 % et 99 % et celle de Ca₂NaK(PO₄)₂ se situe entre 99 % et 1 %.

4. Composition selon l'une quelconque des revendications 1 à 3, dans laquelle le mélange de base se présente sous la forme d'un mélange de poudre.

5. Composition selon l'une quelconque des revendications 1 à 4, contenant en plus au moins l'un des composants suivants :
CaHPO₄·H₂O, CaCO₃, CaSO₄,·CaSO₄·0,5H₂O, CaSO₄·2H₂O,
CaHPO₄, hydroxyapatite nanoparticulaire.

6. Composition selon l'une quelconque des revendications 1 à 5, contenant un mélange de poudre comprenant le mélange de base avec ajout de CaHPO₄·H₂O et de CaCO₃, et par rapport au mélange de poudre, la part en poids de CaHPO₄·H₂O se situe entre 1 % et 30 % et la part en poids de CaO₃ se situe entre 30 % et 1 %.

7. Composition selon l'une quelconque des revendications 1 à 6, contenant un mélange de poudre comprenant le mélange de base avec ajout de CaHPO₄·H₂O, et par rapport au mélange de poudre, la part en poids de CaHPO₄·H₂O se situe entre 1 % et 60 %.

8. Composition selon l'une quelconque des revendications 1 à 7, contenant un mélange de poudre comprenant le mélange de base avec ajout de CaCO₃, et par rapport au mélange de poudre, la part en poids de CaCO₃ se situe entre 1 % et 60 %.

9. Composition selon l'une quelconque des revendications 1 à 8, contenant en plus au moins l'un des composants suivants :
du mannose-6-phosphate, de l'octosulfate de saccharose, de l'octosulfate de saccharose sous forme de sel complexe de sodium ou de potassium, un antibiotique et un agent désinfectant.

10. Composition selon l'une quelconque des revendications 1 à 9, contenant en plus un liquide de conditionnement.

11. Composition selon la revendication 10, dans laquelle le liquide de conditionnement contient de l'eau.

12. Composition selon la revendication 10 ou 11, qui se présente sous la forme d'une pâte.

13. Composition selon l'une quelconque des revendications 10 à 12, dans laquelle le liquide de conditionnement contient une solution aqueuse de mannose-6-phosphate, d'octosulfate de saccharose ou d'un mélange de ceux-ci.

14. Composition selon l'une quelconque des revendications 10 à 13, dans laquelle le liquide de conditionnement contient une solution aqueuse de Na₂HPO₄.

15. Composition selon l'une quelconque des revendications 10 à 14, dans laquelle le liquide de conditionnement contient au moins l'un des composants suivants
du mannose-6-phosphate, de l'octosulfate de saccharose, de l'octosulfate de saccharose sous forme de sel complexe de sodium ou de potassium, du Na₂HPO₄, un antibiotique et un agent désinfectant.

16. Composition selon l'une quelconque des revendications 1 à 15, contenant en plus des protéines bioactives.

17. Composition selon la revendication 16, contenant comme protéines bioactives des facteurs de croissance, des protéines morphogéniques osseuses, des amélogénines de l'émail et/ou des inhibiteurs d'élastase.

18. Utilisation de la composition selon l'une quelconque des revendications 1 à 17 pour la fabrication d'un implant pour réparer ou remplacer des os, des dents ou des parties de ceux-ci.

19. Utilisation selon la revendication 18, dans laquelle la composition sous forme de pâte est appliquée au substrat choisi, modelée et durcie.
